# FASCICULE DE BREVET EUROPEEN

(11) **EP 2 640 767 B1**
(45) Date de publication et mention de la délivrance du brevet: **04.03.2015**
(21) Numéro de dépôt: 11794202.9
(22) Date de dépôt: 14.11.2011
(51) Int. Cl.: C08G 63/08, C08G 63/685, C08G 63/82, C08G 63/81, A61K 9/14, A61K 9/16, A61K 9/20, A61K 9/70, A61K 9/51, A61K 47/34

(54) **PROCÉDÉ DE PRÉPARATION DE POLYMÈRES À ARCHITECTURE VARIÉE ET AMORÇAGE AMIDE**
VERFAHREN ZUR HERSTELLUNG VON POLYMEREN MIT UNTERSCHIEDLICHEN STRUKTUREN UND AMIDINITIERUNG
PROCESS FOR THE PREPARATION OF POLYMERS WITH VARIOUS STRUCTURES AND AMIDE INITIATING

(30) Priorité: 15.11.2010 FR 1004428
(43) Date de publication de la demande: 25.09.2013
(73) Titulaire: IPSEN PHARMA S.A.S., 92100 Boulogne-Billancourt (FR)
(72) Inventeur: BOURISSOU Didier, F-31830 Plaisance Du Touch (FR); MARTIN-VACA Blanca, F-31100 Toulouse (FR); ALBA Aurélie, F-31450 NOUEILLES (FR); CHERIF CHEIKH Roland, E-08860 Castelldefels (Barcelona) (ES); DE SOUSA DELGADO Anne-Paula, E-08750 Molins de Rei (Barcelona) (ES)
(74) Mandataire: Audonnet, Nathalie
(86) Numéro de dépôt international: PCT/FR2011/000602
(87) Numéro de publication internationale: WO 2012/066195

(56) Documents cités:
- EP-A2- 0 879 838
- US-A- 5 514 380
- US-A1- 2007 003 625
- BREITENBACH A., LI Y.X., KISSEL T.: "Branched biodegradable polyesters for parenteral drug delivery systems", JOURNAL OF CONTROLLED RELEASE, vol. 64, 1 janvier 2000 (2000-01-01), pages 167-178, XP002633317, cité dans la demande
- ZHAO W., CUI D., LIU X., CHEN X.: "Facile synthesis od hydroxyl-ended highly stereoregular , star-shaped poly(lactide) from immortal ROP of rac-lactide and kinetics study", MACROMOLECULES, vol. 43, 28 juillet 2010 (2010-07-28), pages 6678-6684, XP002633318,
- COULEMBIER O., KIESEWETTER M.K., MASON A., DUBOIS P., HEDRICK J.L., WAYMOUTH R.M.: "A distinctive organocatalytic approach to complex macromolecular architectures", ANGEWANDTE CHEMIE INTERNATIONAL EDITION, vol. 46, 15 mai 2007 (2007-05-15), pages 4719-4721, XP002633319, cité dans la demande
- BUWALDA S.J., DIJKSTRA P.J., CALUCCI L., FORTE C., FEIJEN J.: "Influence of amide versus ester linkages on the properties of eight-armed PEG-PLA star block copolymer hydrogels", BIOMACROMOLECULES, vol. 11, 25 novembre 2009 (2009-11-25), pages 224-232, XP002633320, cité dans la demande
- KRICHELDORF H R; LOMADZE N; SCHWARZ G: "Cyclic polylactides by imidazole-catalyzed polymerization of L-lactide", MACROMOLECULES, vol. 41, 8 octobre 2008 (2008-10-08), pages 7812-7816, XP002633321,

## Description

### DOMAINE DE L'INVENTION

La présente demande a pour objet un procédé de préparation de polymères d'architectures variées (linéaire et étoile), à base de lactide et/ou de glycolide, ainsi que de nouveaux polymères pouvant éventuellement être obtenus par ce procédé. Ces polymères possèdent des propriétés physico-chimiques intéressantes. Ce procédé est facilement contrôlable et présente une meilleure modulation des polymères et donc de leurs propriétés que les procédés de l'art antérieur.

### ETAT DE LA TECHNIQUE

De nos jours, une attention croissante est portée aux polymères synthétiques pour l'élaboration d'organes artificiels et la formulation de médicaments [Chem. Eng. News 2001, 79 (6), 30]. Les polymères concernés doivent respecter un certain nombre de critères et, en particulier, ils doivent être biocompatibles. Le caractère biodégradable est un avantage supplémentaire si le polymère doit être éliminé après une période appropriée d'implantation dans un organisme. A cet égard, les copolymères à base d'acide lactique et glycolique (PLGA) présentent un très grand intérêt car ils sont sensibles à l'hydrolyse et sont dégradés *in vivo* avec libération de sous-produits non-toxiques. Le champ d'application des PLGA est très vaste (Adv. Mater. 1996, 8, 305 et Chemosphere 2001, 43, 49). Dans le domaine chirurgical, ils sont utilisés pour la synthèse de fils multi-brins, de sutures, d'implants, de prothèses... En pharmacologie, ils permettent l'encapsulation, le transfert et la libération contrôlée de principes actifs. Pour toutes ces applications, un des facteurs clés est la vitesse de dégradation des PLGA qui dépend bien sûr de leur structure (longueur de chaîne, dispersité, proportion, stéréochimie et enchaînement des monomères...).

Afin d'obtenir de nouvelles propriétés, il peut être intéressant de modifier la structure des PLGA. Cependant, les modifications possibles sont très limitées et certaines ont été déjà décrites : masse molaire, tacticité... Un des paramètres n'a été que peu exploré : la modification des extrémités. Il a cependant été décrit que les propriétés physiques et les vitesses de dégradation de PLGA à extrémité ester sont différentes de celles d'une PLGA à extrémité acide (WO200804963). En effet, une nouvelle fonction pourrait apporter des propriétés intéressantes.

La demanderesse a constaté que des PLGA présentant une extrémité amide pourraient être particulièrement intéressants. Or la plupart des procédés actuels ne prennent en compte que des amorceurs à fonction hydroxy (alcool / eau), permettant d'obtenir des PLGA à extrémité ester/acide. Un exemple d'obtention d'une structure polylactide (PLA) par catalyse nucléophile de polymérisation du lactide à partir d'un amorceur de type amine primaire (RNH₂), est décrit dans O. Coulembier, M. K. Kiesewetter, A. Mason, P. Dubois, J. L. Hedrick, R. M. Waymouth, Angew. Chem. Int. Ed. 2007, 46, 4719. A partir d'un poly(éthylèneglycole) fonctionnalisé avec des amines primaires, la polymérisation par ouverture de cycle (ROP ou Ring Opening Polymerization) du lactide catalysée par des carbènes donne accès à des PLA d'architectures complexes. Sur chaque amine primaire, il y a croissance de deux bras PLA. Il n'est donc pas possible par ce procédé de greffer une seule branche sur une amine primaire. J. Liu, L. Liu, Macromolecules 2004, 37, 2674 décrit l'obtention d'un polyester linéaire simple à extrémité amide. Elle est seulement décrite avec des polycaprolactones (PCL) (24 à 48 heures de polymérisation en masse à 160 °C).

Les polymères branchés, parmi lesquels figurent les polymères en étoile, les dendrimères et les polymères hyperbranchés, ont fait l'objet de nombreuses études, de par leurs propriétés rhéologiques et mécaniques intéressantes.

En particulier, les polymères étoilés, ou polymères à architecture étoile, peuvent être utilisés dans l'administration de principes actifs et présentent des profils de libération intéressants. Ce type de polymère est généralement préparé à partir d'amorceurs polyols comportant n fonctions alcools pour conduire à des étoiles à n bras.

En outre, les polymères étoilés présentent des températures de transition vitreuse, ainsi qu'une viscosité à l'état vitreux, légèrement plus faibles que leurs équivalents linéaires. Il en est de même en ce qui concerne leur cristallinité - et donc leur température de fusion - qui est également plus faible que leurs équivalents linéaires. Cependant, la phase cristalline reste de même nature dans les deux architectures.

Un polymère étoile biodégradable (par exemple, le PLGA) aura une vitesse de dégradation initiale beaucoup plus rapide que son équivalent linéaire de même masse. En effet, la vitesse de relargage et de dégradation est à mettre en corrélation avec la structure de la matrice polymère. Il a été montré que par hydrolyse chimique ou enzymatique, les premières coupures de liaisons esters s'effectuent au coeur de l'étoile, proche de l'amorceur, libérant ainsi des polymères linéaires de masses moléculaires plus faibles. Par contre il existe un exemple d'un polymère étoile à coeur PEG lien amide-PLA où les premières coupures se font sur les liaisons esters et les liens amides s'hydrolysent plus tardivement (Biomacromolecules 2010, 11, 224).

Ces différences de propriétés donnent donc accès à des matrices novatrices intéressantes. Par exemple, l'encapsulation de principes actifs dans des polymères étoilés pour des PLGA a été décrite par A. Breitenbach, Y. X. Li, T. Kissel, Journal of Controlled Release 2000, 64, 167.

La polymérisation par ouverture de cycle à partir de complexes métalliques pour la synthèse de polymères à architecture étoile a été décrite dès les années 1990. Les polymères étoile sont principalement préparés en solution ou en masse, avec des catalyseurs métalliques tels que l'octanoate d'étain, même si d'autres systèmes à base de Fe, Zn, Al... ont été rapportés (H.R. Kricheldorf, Polymer for Advanced Technologies 2002, 13, 969 ; A. Finne, A.-C. Albertsson, Biomacromolecules 2002, 3, 684 ; H. R. Kricheldorf, H. Hachmann-Thiessen, G. Schwarz, Biomacromolecules 2004, 5, 492 ; I. Arvanitoyannis, A. Nakayama, E. Psomiadou, N. Kawasaki, N. Yamamoto, Polymer 1996, 37, 651).

La demanderesse a développé un nouveau procédé non-métallique, facilement contrôlable et présentant une meilleure souplesse que les procédés de l'art antérieur.

La demanderesse a également développé de nouveaux polymères linéaires à extrémité amide ou à architecture étoile à coeur amide.

### RESUME DE L'INVENTION

L'invention a donc pour objet un procédé de préparation de polymères linéaires à extrémité amide ou à architecture étoile à coeur amide par ouverture de cycle à base de monomères lactide et glycolide ou d'un monomère lactide, comprenant les étapes consistant à :
(i) faire réagir le ou les monomères en excès avec un amorceur dans un solvant, ledit amorceur étant choisi parmi une amine et un aminoalcool, étant donné que l'amorceur présente au moins une fonction amine primaire ou secondaire,
(ii) ajouter un catalyseur, ledit catalyseur étant une base non nucléophile, et comprenant au moins un atome d'azote de type sp2,
(iii) neutraliser le mélange réactionnel.
   et préférentiellement les étapes consistant à :
(iv) faire réagir le ou les monomères en excès avec un amorceur dans un solvant, ledit amorceur étant choisi parmi une amine et un aminoalcool, étant donné que l'amorceur présente au moins une fonction amine primaire ou secondaire,
(v) ajouter un catalyseur, ledit catalyseur étant une base non nucléophile, et comprenant au moins un atome d'azote neutre de type sp2,
(vi) neutraliser le mélange réactionnel.

De préférence, le monomère est le lactide.

De préférence, les polymères sont préparés à base d'un monomère lactide et d'un monomère glycolide.

De préférence, l'étape (ii) est effectuée après incorporation complète de l'amorceur.

De préférence, le catalyseur basique est choisi parmi :
- la 1,8-diazabicyclo[5.4.0]undec-7-ène (DBU),
- la 1,5-diazabicyclo[4.3.0]non-5-ène (DBN),
- un composé 4-amino-pyridine de formule : 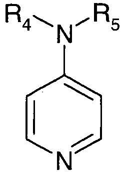 dans laquelle R₄ et R₅ sont indépendamment choisis parmi un atome d'hydrogène ou un radical alkyle en C₁-C₁₂ ; ou bien R₄ et R₅ forment ensemble avec l'atome d'azote qui les porte un hétérocycle saturé ;
- une guanidine cyclique de formule : 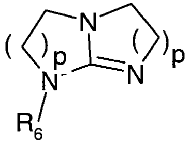 dans laquelle p est 1 ou 2, et R₆ représente un atome d'hydrogène ou un radical alkyle en C₁-C₄ ;
- un phosphazène de formule : 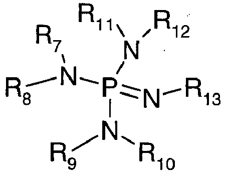
   dans laquelle R7, R10, R11, et R12 représentent indépendamment un radical alkyle en C1 à C6,
   R₈ et R₉ représentent indépendamment un atome d'hydrogène ou un radical alkyle en C1 à C6, ou bien R₈ et R₉ forment ensemble avec les atomes d'azote qui les portent un hétérocycle saturé,
   R₁₃ représente un radical alkyle en C1 à C6.

De préférence, la réaction a lieu dans un solvant organique, de préférence dans un solvant halogéné ou aromatique.

De préférence, le solvant est un solvant halogéné, de préférence, le solvant est le dichlorométhane.

De préférence, l'amorceur est une amine.

De préférence, l'amorceur est un aminoalcool.

De préférence, la température de réaction est de 0 à 150°C, de préférence de 20 à 45°C.

Ce procédé a l'avantage de permettre l'incorporation complète de l'amorceur dans les chaînes polymères en tant qu'extrémité amide et donc de conduire à une très bonne efficacité d'amorçage.

L'invention a également pour objet de nouveaux polymères de formule I : 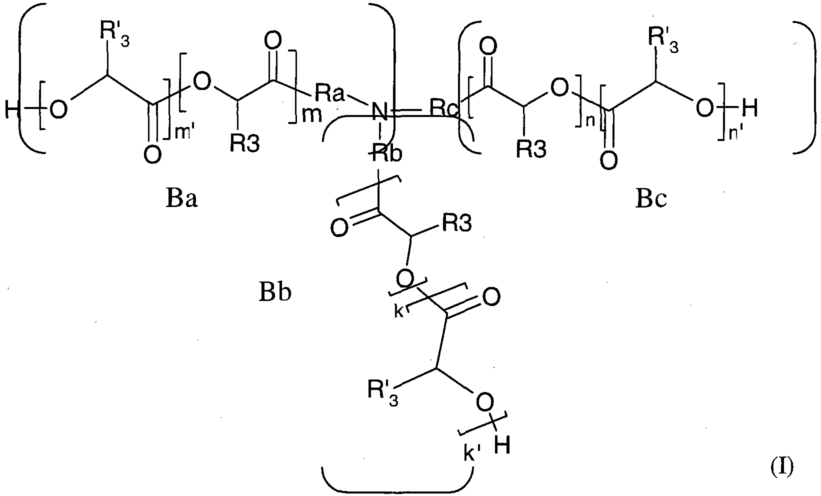 dans laquelle
n, n', m, m', k et k' représentent indépendamment un entier de 0 à 12,
Ra représente une liaison covalente, un radical alkyle en C5 à C14 linéaire ou ramifié, un radical alkylamino, un radical alkyloxy, un radical aryle ou aralkyle, étant entendu que si Ra est un radical aryle ou aralkyle alors m et m' sont nuls,
Rb représente une liaison covalente, un radical alkyle en C5 à C14 linéaire ou ramifié, un radical alkylamino ou un radical alkyloxy,
Rc représente une liaison covalente, un radical alkyle en C5 à C14 linéaire ou ramifié, un radical alkylamino, ou un radical alkyloxy,
R3 représente un atome d'hydrogène et R'3 représente un radical alkyle, étant entendu qu'au moins un des n', m' et k' est différent de zéro ;
ou bien R'3 représente un atome d'hydrogène et R3 représente un radical alkyle, étant entendu qu'au moins un des n, m et k est différent de zéro ;
et étant entendu que :
- au plus une des branches Ba, Bb et Bc représente un atome d'hydrogène
- si une des branches Ba, Bb et Bc représente l'atome d'hydrogène, alors au moins une des deux autres branches est liée à l'atome d'azote par un radical alkylamino.

De préférence, R3 représente un radical alkyle, et n', m' et k' valent zéro.

De préférence, une des branches Ba, Bb et Bc représente l'atome d'hydrogène.

De préférence, au moins un des Ra, Rb et Rc représente un radical alkylamino.

De préférence, au moins un des Ra, Rb et Rc représente un radical alkyloxy.

L'invention a également pour objet une composition pharmaceutique comprenant au moins un polymère selon l'invention.

### BREVE DESCRIPTION DES FIGURES

La figure 1 représente la photographie au microscope électronique du polymère à extrémité CO-NCH3-alkylC12 (exemple 8).
La figure 2 représente la photographie au microscope électronique du polymère à extrémité CO-NH-alkylC12 (exemple 1).

### DESCRITION DETAILLEE DE MODES DE REALISATION DE L'INVENTION

L'invention a donc pour objet un procédé de préparation de polymères linéaires à extrémité amide ou à architecture étoile à coeur amide.

Par polymère étoile, on entend un polymère présentant un seul point de ramification d'où émanent plusieurs chaînes linéaires (ramifications). Par « coeur amide », on entend que le point de ramification est un atome d'azote et qu'au moins une des chaînes linéaires comporte au moins un autre atome d'azote (au « coeur » du polymère) lié à un radical -C(=O)- afin de former une fonction amide. Sur la ou les chaînes linéaires comprenant la fonction amide, il y a au plus 10 atomes successifs qui séparent l'atome d'azote point de ramification de l'atome d'azote de la fonction amide, de préférence, au plus 5 atomes, de manière plus préférée, au plus 3 atomes, de manière encore plus préférée, au plus 2 atomes.

Par exemple, le polymère 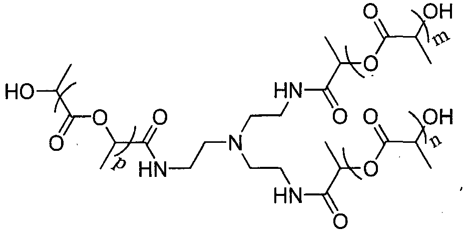 est un polymère à coeur amide : le point de ramification d'où émanent trois chaînes linéaires est un atome d'azote ; deux atomes séparent l'atome d'azote d'une fonction amide de l'atome d'azote point de ramification.

Par polymères linéaires à extrémité amide, on entend un polymère linéaire ayant une de ses deux extrémités de type amide non-substitué, N-monosubstitué ou N,N-disubstitué. Par exemple, on entend un polymère linéaire ayant une extrémité -C(=O)-NH-C₁₂H₂₅.

La réaction de polymérisation est de type ouverture de cycle. La polymérisation par ouverture de cycle est une polymérisation par addition. Elle peut se schématiser de la manière suivante : 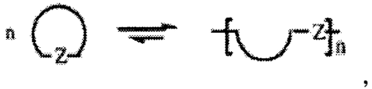 avec n le nombre de monomères.

La réaction est réalisée à partir d'un monomère lactide et d'un monomère glycolide, ou bien à partir d'un monomère lactide seul. Selon une variante, le monomère est le lactide. Selon une autre variante, la réaction est une co-polymération et la réaction est réalisée à partir de lactide et de glycolide.

Le procédé comprend une première étape (i) consistant à faire réagir le ou les monomères avec un amorceur dans un solvant. Le ou les monomères doivent être en excès par rapport à l'amorceur, de préférence, de 1/1 à 100/1, de manière plus préférée, de 1/1 à 30/1, de manière encore plus préférée de 1/1 à 6/1.

L'amorceur est choisi parmi une amine et un aminoalcool.

Par amine, on entend tout composé comportant au moins une fonction amine primaire, secondaire ou tertiaire. On entend par exemple les alkylamines, les diaminoalkyles ou les triaminoalkyles. Par exemple, on entend la tris(2-aminoéthyl)amine.

Par aminoalcool, on entend tout composé comportant au moins une fonction amine primaire, secondaire ou tertiaire et au moins une fonction -OH. Par exemple, on entend la diéthanolamine.

Il est entendu que l'amorceur présente au moins une fonction amine primaire ou secondaire.

Le procédé comprend une deuxième étape (ii) consistant à ajouter un catalyseur.

De préférence, l'étape (ii) est effectuée après que tout l'amorceur ait été incorporé dans l'étape (i), c'est-à-dire qu'il ne reste plus d'amorceur dans le mélange réactionnel.

Il ne reste plus d'amorceur dans le mélange réactionnel lorsque la réaction entre l'amorceur additionné et la quantité stoechiométrique de lactide est finie. La réaction peut être suivie par RMN proton, et dans ce cas, le catalyseur est additionné lors qu'on ne voit plus les signaux d'amorceur.

Par exemple, l'étape (ii) est effectuée après une durée comprise entre 5 et 30 minutes après le début de l'étape (i), de préférence, de 10 à 20 minutes.

Ceci permet d'obtenir une excellente efficacité d'amorçage.

Le catalyseur est une base non nucléophile, de préférence une base forte non nucléophile. Le catalyseur comprend au moins un atome d'azote de type sp2, c'est-à-dire que l'azote est de type =N-, c'est-à-dire lié d'un côté (à un premier atome adjacent) par une liaison double et de l'autre côté (à un second atome adjacent) par une liaison simple. De préférence, le catalyseur comprend au moins un atome d'azote neutre de type sp2. Le catalyseur, base non nucléophile, réagit de préférence en tant que base de Bronsted et non en tant que nucléophile.

Le catalyseur est une base non nucléophile qui peut être choisie parmi les dérivés diazacycloalcène ; les dérivés amino-pyridine tels que les dérivés de 4-amino-pyridines ; les dérivés de guanidine cyclique ; ou bien les dérivés de phosphazène.

Le catalyseur est une base non nucléophile qui peut être préférentiellement choisie parmi :
- les dérivés diazacycloalcène tels que les diazabicycloundécène, les diazabicyclononène ;
- les dérivés 4-amino-pyridine tels que les dérivés d'4-amino-pyridines de formule : 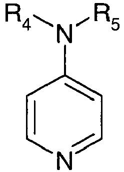 dans laquelle R4 et R5 sont indépendamment choisis parmi un atome d'hydrogène ou un radical alkyle en C1-C12 ; ou bien R4 et R5 forment ensemble avec l'atome d'azote qui les porte un hétérocycle saturé ;
- les dérivés de guanidine cyclique de formule 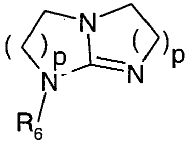 dans laquelle p est 1 ou 2, et R6 représente un atome d'hydrogène ou un radical alkyle en C1-C4 ;
- ou bien les dérivés de phosphazène de formule : 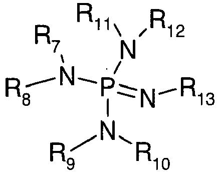
   dans laquelle R7, R10, R11, et R12 représentent indépendamment un radical alkyle en C1 à C6,
   R8 et R9 représentent indépendamment un atome d'hydrogène ou un radical alkyle en C1 à C6, ou bien R8 et R9 forment ensemble avec les atomes d'azote qui les portent un hétérocycle saturé,
   R13 représente un radical alkyle en C1 à C6.

Le catalyseur est une base non nucléophile qui peut être préférentiellement choisie parmi : la 1,8-diazabicyclo[5.4.0]undec-7-ène (ou DBU), la 1,5-diazabicyclo[4.3.0]non-5-ène (DBN); la N',N'-diméthylamino-4-pyridine (ou DMAP), 1,5,7-triazabicyclo-[4.4.0]dec-5-ène (TBD), le 2-tert-butylimino-2-diéthylamino-1,3-diméthylperhydro-1,3,2-diazaphosphorine (ou BEMP).

Par exemple, le catalyseur est la DBU (la 1,8-diazabicyclo[5.4.0]undec-7-ène).

De préférence, le catalyseur est un composé 4-amino-pyridine de formule : 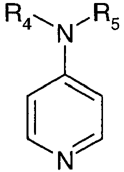 dans laquelle R4 et R5 sont indépendamment choisis parmi un atome d'hydrogène ou un radical alkyle en C1-C12 ; ou bien R4 et R5 forment ensemble avec l'atome d'azote qui les porte un hétérocycle saturé. Par un composé 4-amino-pyridine de formule : 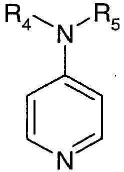 on entend par exemple la N',N'-diméthylamino-4-pyridine (ou DMAP).

De préférence, le catalyseur est une guanidine cyclique de formule : 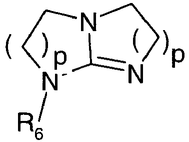 dans laquelle p est 1 ou 2, et R6 représente un atome d'hydrogène ou un radical alkyle en C1-C4. Comme guanidine cyclique, on entend par exemple la 1,5,7-triazabicyclo-[4.4.0]dec-5-ène (TBD).

De préférence, le catalyseur est un phosphazène, et préférenciellement un monophosphazène. De préférence, le catalyseur est un monophosphazène de formule : 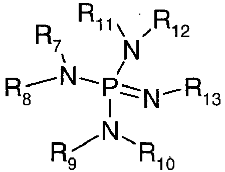
dans laquelle R7, R10, R11, et R12 représentent indépendamment un radical alkyle en C1 à C6,
R8 et R9 représentent indépendamment un atome d'hydrogène ou un radical alkyle en C1 à C6, ou bien R8 et R9 forment ensemble avec les atomes d'azote qui les portent un hétérocycle saturé,
R13 représente un radical alkyle en C1 à C6.

Comme composé monophosphazène tel que défini ci-dessus, on entend par exemple le 2-tert-butylimino-2-diéthylamino-1,3-diméthylperhydro-1,3,2-diazaphosphorine (BEMP).

De manière préférée, le rapport initial concentration en fonction NH2 de l'amorceur sur concentration en catalyseur est de 1 à 1000, de manière plus préférée de 2 à 500, de manière encore plus préférée de 10 à 100.

Le procédé comprend une troisième étape (iii) consistant à neutraliser le mélange réactionnel. La neutralisation peut être effectuée par tout moyen connu de l'homme du métier. Par exemple, la neutralisation est effectuée par l'ajout d'un acide, ou d'une résine acide telle que l'amberlyste ™ A15.

La réaction a lieu dans un solvant. Le terme « solvant » signifie ici un solvant unique ou un mélange de solvants. De préférence, le solvant est choisi de telle sorte que le polymère formé y soit soluble. De préférence, le solvant est choisi parmi les solvants halogénés, les éthers cycliques et les solvants aromatiques. Par exemple, le solvant est choisi parmi le dichlorométhane, le dichloroéthane, le tétrahydrofurane (THF) et le toluène. De préférence, le solvant est le dichlorométhane.

De préférence, la réaction est réalisée à une température comprise entre la température ambiante, c'est-à-dire environ 25°C, et la température d'ébullition du solvant choisi. La température de réaction est choisie de manière à être inférieure à la température de dégradation du polymère formé. Par exemple, la température est de 0 à 150°C. De préférence, la température est de 10 à 90°C. De préférence encore, la température est de 20 à 45°C, de préférence de 20 à 30°C. Par exemple, la réaction est réalisée à température ambiante.

Alternativement, l'étape (i) est réalisée par chauffage, de préférence à une température comprise entre 50 et 80°C, de préférence par chauffage à reflux. Ceci est préféré lorsque l'amorceur est une amine secondaire.

Dans cette alternative, l'étape (ii) est réalisée de préférence à une température de 15 à 35°C, de préférence, à température ambiante.

De préférence, la réaction est arrêtée par l'étape (iii) une fois le degré de polymérisation souhaité obtenu. Par exemple, la réaction est arrêtée quand la consommation du monomère initial est de 90 à 100%. De manière préférée la réaction est arrêtée quand la consommation du monomère initial est supérieure à 96 %.

Le procédé selon l'invention présente de nombreux avantages. Notamment, le procédé permet une grande sélectivité. Ce procédé a l'avantage de permettre l'incorporation complète de l'amorceur dans les chaînes polymères en tant qu'extrémité amide et donc de conduire à une très bonne efficacité d'amorçage. Il est possible grâce à ce procédé d'obtenir des polymères très variés, présentant des propriétés facilement modulables. Il est possible d'obtenir un polymère à 1, 2 ou 3 branches à extrémité amide.

De préférence, le polymère obtenu est un polymère à une branche à extrémité amide. De préférence, le polymère obtenu est un polymère à deux branches à extrémité amide. De préférence, le polymère obtenu est un polymère à trois branches à extrémité amide.

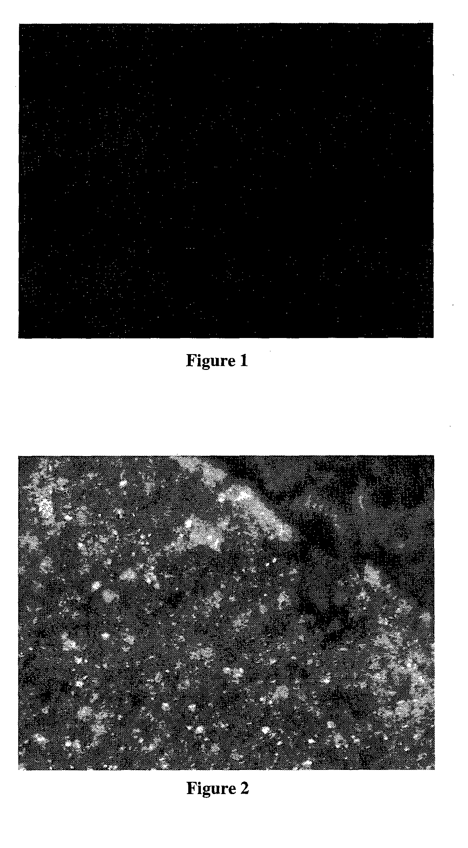

Les figures 1 et 2 montrent ces différences de propriétés.

La figure 1 représente la photographie sous lumière polarisée au microscope électronique du polymère à extrémité CO-NCH3-alkylC12 (exemple 8). La photographie, noire, montre que le polymère est amorphe.

La figure 2 représente la photographie sous lumière polarisée au microscope électronique du polymère à extrémité CO-NH-alkylC12 (exemple 1). Le polymère est cristallin.

L'invention concerne également un nouveau polymère de formule I : 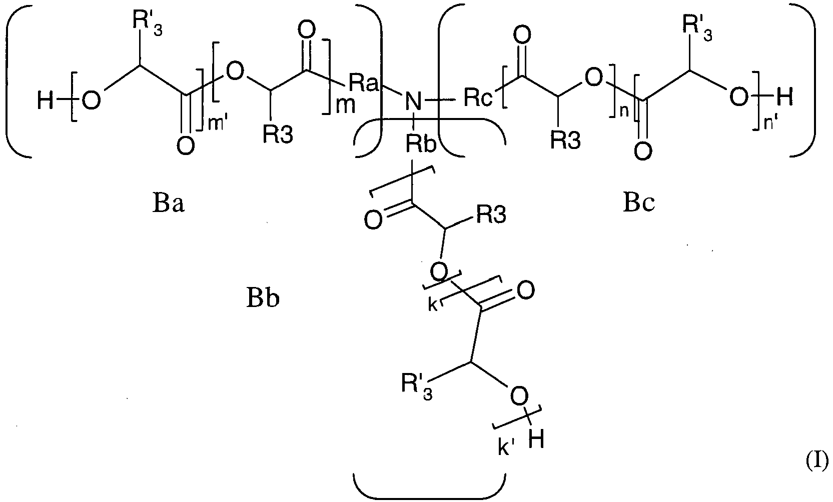 dans laquelle
n, n', m, m', k et k' représentent indépendamment un entier de 0 à 12,
Ra représente une liaison covalente, un radical alkyle en C5 à C14 linéaire ou ramifié, un radical alkylamino, un radical alkyloxy, un radical aryle ou aralkyle, étant entendu que si Ra est un radical aryle ou aralkyle alors m et m' sont nuls,
Rb représente une liaison covalente, un radical alkyle en C5 à C14 linéaire ou ramifié, un radical alkylamino ou un radical alkyloxy,
Rc représente une liaison covalente, un radical alkyle en C5 à C14 linéaire ou ramifié, un radical alkylamino, ou un radical alkyloxy,
R3 représente un atome d'hydrogène et R'3 représente un radical alkyle, étant entendu qu'au moins un des n', m' et k' est différent de zéro ;
ou bien R'3 représente un atome d'hydrogène et R3 représente un radical alkyle, étant entendu qu'au moins un des n, m et k est différent de zéro ;
et étant entendu que :
- au plus une des branches Ba, Bb et Bc représente un atome d'hydrogène
- si une des branches Ba, Bb et Bc représente l'atome d'hydrogène, alors au moins une des deux autres branches est liée à l'atome d'azote par un radical alkylamino.

Lorsqu'il n'est pas donné plus de précisions, le terme alkyle au sens de la présente invention représente un radical alkyl linéaire ou ramifié comprenant entre 1 et 12 atomes de carbones tels que les radicaux méthyle, éthyle, propyle, isopropyle, butyle, isobutyle, sec-butyle et tert-butyle, pentyle ou amyle, isopentyle, neopentyle, hexyle ou isohexyle, heptyle, octyle, nonyle, décyle, undécyle ou dodécyle. Il est entendu que dans la présente demande le radical alkyl peut être de type CnH2n, et présenter deux points de liaisons, en début et fin de chaîne (appelé aussi alkanediyl). De manière préférentielle le radical alkyle sera un radical (C1-C6)alkyle, c'est-à-dire représentant un radical alkyle ayant de 1 à 6 atomes de carbone tel que défini ci-dessus, ou un radical (C1-C4)alkyle représentant un radical alkyle ayant de 1 à 4 atomes de carbone tels que par exemple les radicaux méthyle, éthyle, propyle, isopropyle, butyle, isobutyle, sec-butyle et tert-butyle.

Le terme alkyle dans les expressions alkyloxy (ou alkoxy), alkylamino, dialkylamino, aralkyle représente un radical alkyle tel que défini ci-dessus.

Plus particulièrement, par alkylamino, on entend un radical alkyle dont au moins l'un des atomes d'hydrogène est remplacé par une fonction amine, de préférence, on entend un radical alkyle dont au moins l'un des atomes d'hydrogène terminal, c'est-à-dire à une extrémité de la chaîne alkyle, est remplacé par une fonction amine comme par exemple, et préférentiellement un radical -(CH2)2-NH, ou un radical -(CH2)3-NH.

Plus particulièrement, par alkoxy, on entend un radical alkyle dont au moins l'un des atomes d'hydrogène terminal, c'est-à-dire à une extrémité de la chaîne alkyle, est remplacé par un atome d'oxygène comme par exemple, et préférentiellement un radical -(CH2)2-O-, ou un radical -(CH2)3-O-.

Au sens de la présente invention, les radicaux aryles peuvent être de type mono ou polycycliques aromatiques. Les radicaux aryles monocycliques peuvent être choisis parmi les radicaux phényles, tolyles, xylyles, mésityles, cuményles et de préférence phényles. Les radicaux aryles polycycliques peuvent être choisis parmi les radicaux naphtyle, anthryle, phénanthryle, fluorényle. Ils peuvent être optionnellement substitués par un ou plusieurs radicaux identiques ou différents tels que alkyle, haloalkyle, alkoxy, alkoxycarbonyle, alkylcarbonyloxy, halo, cyano, nitro, aryle, aryloxy, aryloxycarbonyl, ou arylcarbonyloxy.

Le terme aryle dans l'expression aralkyle représente un radical aryle tel que défini ci-dessus. Par exemple, on entend par aralkyle un radical benzyle.

Par hétérocycle saturé, lorsqu'il n'est pas donné plus de précision, on entend un radical cyclique carboné saturé comprenant au moins un hétéroatome choisi parmi N, O et S, tel que l'oxiranne, l'aziridine, l'azétidine, la pipéridine. De préférence, l'hétérocycle saturé comporte de 3 à 7 chaînons, de préférence de 3 à 6 chaînons, de préférence de 4 à 6 chaînons, de manière plus préférée de 5 à 6 chaînons.

Par composé diazacycloalcène, on entend un composé bicyclique condensé comprenant 2 atomes d'azote et au moins une double liaison.

L'invention a également pour objet une composition pharmaceutique comprenant au moins un polymère selon l'invention.

A moins qu'ils ne soient définis d'une autre manière, tous les termes techniques et scientifiques utilisés dans la présente demande ont la même signification que celle couramment comprise par un spécialiste ordinaire du domaine auquel appartient l'invention.

Les exemples suivants sont présentés pour illustrer l'invention et ne doivent en aucun cas être considérés comme une limite à la portée de l'invention.

### EXEMPLES

### Exemples 1 et 2 :

### Mode opératoire général

Le lactide (LA) et l'amorceur aminé (1 équivalent) sont dissous dans du dichlorométhane fraîchement distillé ([LA]₀ = 1 mol.L⁻¹). Le milieu réactionnel est agité 20 minutes à T = 26°C (jusqu'à incorporation complète de l'amine, contrôlée par spectroscopie RMN ¹H) puis la DBU (0,01 équivalent) est ajoutée et le milieu réactionnel est agité à T = 26 °C, entre 3 et 10 minutes, jusqu'à consommation totale du lactide (également contrôlée par spectroscopie RMN ¹H).

10 équivalents (par rapport à la DBU) de résine Amberlyst A15 (∼5 méq/g), préalablement lavée et séchée, sont ajoutés afin d'éliminer le catalyseur. Le milieu réactionnel est agité 10 minutes puis filtré. 5 équivalents de résine Amberlyst A15 sont de nouveau ajoutés au milieu réactionnel qui est agité 10 minutes puis filtré. Le solvant de réaction est ensuite évaporé sous vide puis le polymère obtenu est séché sous vide pendant 48 heures à 50°C pour les polyesters linéaires et à 60°C pour les étoiles.

### Exemple 1 : polymère amorcé avec la dodécylamine et de DP=3.5

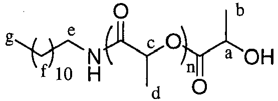

¹H RMN (*δ*, CDCl₃, 300.1 MHz) : 6.15 (1H, br s, NH), 5.20-5.13 (6.2H, m, CH_{c}), 4.35 (1H, q, *J* = 6.7 Hz, CHₐ), 3.20 (2H, m, CH₂ₑ), 1.60-1.54 (16H, m, CH_{3d}), 1.48 (8H, m, CH_{3d}, CH_{3b} et CH_{2f}), 1.25 (18H, m, CH_{2f}), 0.88 (3H, t, *J* = 6.7 Hz, CH_{3g}) ppm.
¹³C RMN (*δ*, CDCl₃, 75.5 MHz) : 169.6 (CO), 71.8 (CH) 69.8-68.5 (CH), 66.7 (CH-OH), 39.4 (CH₂N), 31.9 (CH₂), 29.7-29.6 (CH₂), 29.4-29.3 (CH₂), 26.9-26.8 (CH₂), 22.7 (CH₂), 21.4 (CH₃), 20.5 (CH₃), 17.8 (CH₃), 16.8-16.7 (CH₃), 14.1 (CH₃) ppm.
DP_{RMN} = 3.6
% amine incorporée > 99 %
SEC (THF): Mₙ = 1056, M_{w}/Mₙ = 1.14.

### Exemple 2 : polymère amorcé avec la diéthanolamine en présence de 6 équivalents de D,L-lactide

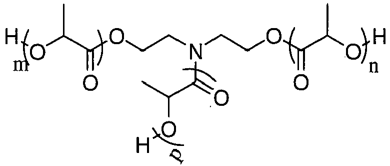

¹H RMN (*δ*, CDCl₃, 300.1 MHz) : 5.21-5.14 (8.3H, m, CH), 4.35-4.23 (7H, m, CH-OH et CH₂-O), 2.87 (2H, m, CH₂-N), 1.60-1.50 (23, m, CH₃), 1.46-1.48 (9.1H, m, CH₃-OH) ppm.
¹³C RMN (*δ*, CDCl₃, 75.5 MHz) : 169.6 (CO), 69.4-69.0 (CH), 66.6 (CH), 64.6 (CH₂), 47.4 (CH₂), 20.4 (CH₃), 16.6 (CH₃) ppm.
DP_{RMN} = 5.7
% amine incorporée > 99 %
SEC (THF): Mₙ = 1134, M_{w}/Mₙ = 1.25.

### Exemple 3 : synthèse d'un D,L-PLA de DP=30 amorcé par la dodécylamine

Le D,L-lactide (30 équivalents) et la dodécylamine (1 équivalent) sont dissous dans du dichlorométhane fraîchement distillé ([LA]₀ = 1 mol.L⁻¹). Le milieu réactionnel est agité 20 minutes à température ambiante puis la DBU (0.05 équivalent) est ajoutée. Le mélange est vivement agité à température ambiante jusqu'à consommation totale du lactide, contrôlée par spectroscopie RMN ¹H. Au bout de 5 minutes, le milieu réactionnel est neutralisé par ajout d'acide benzoïque. La phase organique peut être lavée avec de l'eau, puis une solution de NaHCO₃ saturée, et enfin avec une solution saturée de NaCl afin d'éliminer le catalyseur. La phase organique est ensuite séchée sur Na₂SO₄, filtrée et évaporée pour conduire au polymère. 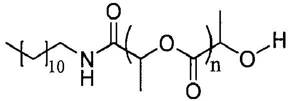 ¹H RMN (*δ*, CDCl₃, 300.1 MHz) : 6.19 (1H, br s, NH), 5.17-5.14 (57H, m, CH), 4.35 (1H, q, CH), 3.27-3.05 (2H, m, CH₂), 1.58-1.53 (178H, m, CH), 1.24 (18H, br s, CH₂), 0.87 (3H, t, CH₃) ppm.
DP_{RMN} = 29
% amine incorporée > 99 %
SEC (THF): M_{w} = 6608, M_{w}/Mₙ = 1.18

### Exemple 4 : Synthèse d'un copolymère PLGA 80/20 de DP=3,5 amorcé par la dodécylamine

Le lactide (2.8 équivalents), le glycolide (0.7 équivalents) et la dodécylamine (1 équivalent) sont dissous dans du dichlorométhane fraîchement distillé ([LA]₀ = 1 mol.L⁻¹). Le milieu réactionnel est agité 25 minutes à température ambiante puis la DBU (0.05 équivalent) est ajoutée et le milieu réactionnel est vivement agité. Au bout de 3 minutes, le milieu réactionnel est neutralisé par ajout d'acide benzoïque et la consommation totale du monomère est vérifiée par spectroscopie RMN ¹H. 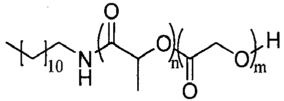 ¹H RMN (*δ*, CDCl₃, 300.1 MHz) : 6.28 (1H, br s, NH), 5.25-5.15 (4.5H, m, CHₚₒₗ), 4.90-4.67 (3.0H, m, CH₂ₚₒₗ), 4.35 (1H, m, CH), 3.32-3.21 (2H, m, CH₂), 1.60-1.48 (18.4H, m, CH₂ et CH₃), 1.24 (18h, br s, CH₂), 0.87 (3H, t, CH₃) ppm.
DP_{RMN} = 3.5
% amine incorporée : > 99%
Ratio lactide/glycolide = 79/21 (par RMN ¹H)
SEC (THF): M_{w} = 1008, M_{w}/Mₙ = 1.19

### Exemple 5 : Synthèse d'un L-PLA de DP=30 amorcé par la benzylamine

Le L-lactide (30 équivalents) et la benzylamine (1 équivalent) sont dissous dans du dichlorométhane fraîchement distillé ([LA]₀ = 1 mol.L⁻¹). Le milieu réactionnel est agité 30 minutes à température ambiante puis la DBU (0.06 équivalent) est ajoutée. Le mélange est vivement agité. Au bout de 5 minutes, le milieu réactionnel est neutralisé par ajout d'acide benzoïque et la consommation totale du monomère est vérifiée par spectroscopie RMN ¹H. 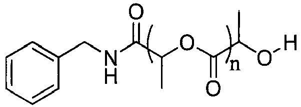 ¹H RMN (*δ*, CDCl₃, 300.1 MHz) : 7.42-7.27 (5H, m, CH), 6.62 (1H, br s, NH), 5.25-5.15 (52H, q, *J*=7.1 Hz, CHₚₒₗ), 4.48 (2H, m, CH₂), 4.39 (1H, q, *J*=6.9 Hz, CH), 1.61-1.47 (160H, m, CH₃) ppm.
¹³C RMN (*δ*, CDCl₃, 75.5 MHz) : 169.6 (CO), 129.2 (C), 128.6 (CH), 127.7 (CH), 127.5 (CH), 71.8 (CH), 69.8 (CH), 69.0 (CH), 66.7 (CHOH), 43.2 (CH₂), 20.5 (CH₃), 17.8 (CH₃), 16.6 (CH₃ₚₒₗ) ppm. Le CO amide n'est pas observé.
DP_{RMN} = 26
% amine incorporée : > 99%
SEC (THF): M_{w} = 8114, M_{w}/Mₙ = 1.12

### Exemple 6 : Synthèse d'un L-PLA de DP=30 amorcé par la 1,3 propanediamine

Le L-lactide (30 équivalents) et la 1,3 propanediamine (1 équivalent) sont dissous dans du dichlorométhane fraîchement distillé ([LA]₀ = 1 mol.L⁻¹). Le milieu réactionnel est agité 20 minutes à température ambiante puis la DBU (0.02 équivalent) est ajoutée et le mélange est vivement agité. Au bout de 3 minutes, le milieu réactionnel est neutralisé par ajout d'acide benzoïque et la consommation totale du monomère est vérifiée par spectroscopie RMN ¹H. 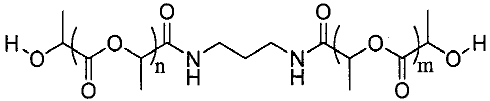 ¹H RMN (*δ*, CDCl₃, 300.1 MHz) : 6.81 (2H, br s, NH), 5.16 (63H, q, *J*=7.1 Hz, CHₚₒₗ), 4.37 (2H, q, *J*=6.9 Hz, CH), 3.48 (2H, m, CH₂), 3.25 (4H, m, CH₂), 1.58 (195H, d, *J*=7.1 Hz, CH₃) ppm.
¹³C RMN (*δ*, CDCl₃, 75.5 MHz) : 169.6 (CO), 71.7 (CH), 69.1 (CH), 66.7 (CHOH), 35.5 (CH₂), 20.5 (CH₃), 17.8 (CH₃), 16.7 (CH₃ₚₒₗ) ppm. Le CO amide et le CH₂ central ne sont pas observé.
DP_{RMN} = 32.5
% amine incorporée : > 99%
SEC (THF): M_{w} = 8709, M_{w}/Mₙ = 1.12

### Exemple 7 : Synthèse d'un D,L-PLA de DP=30 amorcé par la tris(2-aminoéthyl)amine

Le *D,L*-lactide (30 équivalents) et la tris(2-aminoéthyl)amine (1 équivalent) sont dissous dans du dichlorométhane fraîchement distillé ([LA]₀ = 1 mol.L⁻¹). Le milieu réactionnel est agité 30 minutes à température ambiante puis la DBU (0.1 équivalent) est ajoutée et le mélange est vivement agité. Au bout de 10 minutes, le milieu réactionnel est neutralisé par ajout d'acide benzoïque et la consommation totale du monomère est vérifiée par spectroscopie RMN ¹H. 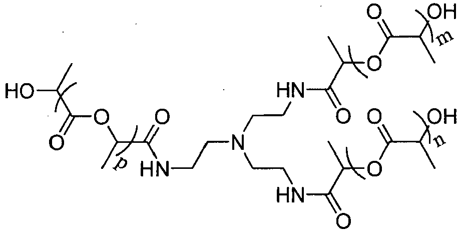 ¹H RMN (*δ*, CDCl₃, 300.1 MHz) : 6.91-6.71 (3H, br s, NH), 5.20-5.14 (72H, m, CHₚₒₗ), 4.35 (3H, q, *J* = 6.9 Hz, CH), 3.44-3.04 (6H, m, CH₂), 2.64-2.48 (6H, m, CH₂), 1.61-1.48 (225H, m, CH₃) ppm.
¹³C RMN (*δ*, CDCl₃, 75.5 MHz) : 175.1 (CO), 169.6 (CO), 69.1 (CH), 69.0 (CH), 66.7 (CHOH), 54.4 (CH₂), 38.0 (CH₂), 20.5 (CH₃), 17.6 (CH₃), 16.6 (CH₃) ppm.
DP_{RMN} = 37.5
% amine incorporée > 99 %
SEC (THF): M_{w} = 8514, M_{w}/Mₙ = 1.09

### Exemple 8 : Synthèse d'un D,L-PLA de DP=3.5 amorcé par la N-méthyl dodécylamine, dans le THF à 70°C

Le D,L-lactidé (3.2 équivalents) et la N-méthyldodécylamine (1 équivalent) sont dissous dans du THF fraîchement distillé ([LA]₀ = 1 mol.L⁻¹). Le milieu réactionnel est agité 3 heures à T = 70°C puis la DBU (0.05 équivalent) est ajoutée et le mélange est vivement agité à température ambiante. Au bout de 4 minutes, le milieu réactionnel est neutralisé par ajout d'acide benzoïque (1.5 éq, 21 mg). Le THF est évaporé et le milieu réactionnel est repris dans 70 mL de dichlorométhane. La phase organique est lavée 2 fois avec une solution saturée de NaHCO₃, une fois avec de l'eau, une fois avec une solution saturée de sel, puis séchée sur Na₂SO₄, filtrée et évaporée. Le polymère obtenu est séché 48h à 50°C puis stocké sous argon. 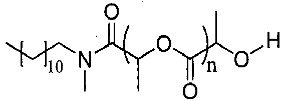 ¹H RMN (*δ*, CDCl₃, 300.1 MHz) : 5.37 (1H, m, CH), 5.20 (4.8H, m, CHₚₒₗ), 4.37 (1H, m, CH) 3.45 (0.6H, m, CH₂), 3.23 (1.4H, m, CH₂), 2.99-2.91 (3H, m, CH₃), 1.59-1.48 (22H, m, CH₃), 1.26 (18H, br s, CH₂), 0.88 (3H, m, CH₃) ppm.
¹³C RMN (*δ*, CDCl₃, 75.5 MHz) : 175.1 (CO), 169.6 (CO), 169.4 (CO), 69.0 (CH), 67.8 (CH), 66.7 (CHOH), 49.6 (CH₂), 48.2 (CH₂), 34.7 (CH₃), 33.8 (CH₃), 31.9 (CH₂), 29.6 (CH₂), 29.5 (CH₂), 29.3 (CH₂), 28.3 (CH₂), 27.0 (CH₂), 26.8 (CH₂), 26.7 (CH₂), 22.7 (CH₂), 20.5 (CH₃), 17.2 (CH₃), 16.6 (CH₃), 16.3 (CH₃), 14.1 (CH₃) ppm.
DP_{RMN} = 3.4
% amine incorporée > 96 %
Rendement = 75%
SEC (THF): M_{w} = 1162, M_{w}/Mₙ = 1.24

### EXEMPLES COMPARATIFS

Afin de démontrer les avantages du procédé selon l'invention, des essais comparatifs ont été réalisés.

Un premier polymère a été synthétisé à partir d'un amorceur alcool (1-dodécanol). Les autres polymères synthétisés à partir d'un amorceur amine, selon l'invention, sont ceux des exemples 1 et 8.

Les propriétés suivantes ont été obtenues :

| | Mm moyenne (g/mol) | Tg (°C) | Apparence |
|---|---|---|---|
| Comparatif (ester) | 1324 | -30 | Fluide |
| Exemple 1 (amine primaire) | 1372 | -25 | Pâteux (cristallin) |
| Exemple 8 (amine secondaire) | 1067 | -36 | Fluide (Amorphe) |

Cette modulation des propriétés du polymère n'est pas possible avec un PLA/PLGA amorcé avec un alcool. L'amorçage avec les amines permet de moduler la fluidité et la cristallinité en fonction du type d'amine utilisé. En effet, il y a création possible de liaison(s) hydrogène entre les chaînes, ce qui n'est pas possible avec l'amorçage alcool.

## Revendications

1. Procédé de préparation de polymères linéaires à extrémité amide ou à architecture étoile à coeur amide par ouverture de cycle à base de monomères lactide et glycolide ou d'un monomère lactide, comprenant les étapes consistant à :
(i) faire réagir le ou les monomères en excès avec un amorceur dans un solvant, ledit amorceur étant choisi parmi une amine et un aminoalcool, étant donné que l'amorceur présente au moins une fonction amine primaire ou secondaire,
(ii) ajouter un catalyseur, ledit catalyseur étant une base non nucléophile, et comprenant au moins un atome d'azote neutre de type sp²,
(iii) neutraliser le mélange réactionnel.

2. Procédé de préparation de polymères selon la revendication 1, dans lequel le monomère est le lactide.

3. Procédé de préparation de polymères selon la revendication 1, dans lequel les polymères sont préparés à base d'un monomère lactide et d'un monomère glycolide.

4. Procédé de préparation de polymères selon l'une des revendications 1 à 3, dans lequel l'étape (ii) est effectuée après incorporation complète de l'amorceur.

5. Procédé de préparation de polymères selon l'une des revendications 1 à 4, dans lequel le catalyseur basique est choisi parmi :
- la 1,8-diazabicyclo[5.4.0]undec-7-ène,
- la 1,5-diazabicyclo[4.3.0]non-5-ène,
- un composé 4-amino-pyridine de formule : 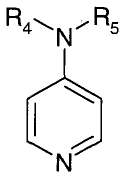 dans laquelle R₄ et R₅ sont indépendamment choisis parmi un atome d'hydrogène ou un radical alkyle en C₁-C₁₂ ; ou bien R₄ et R₅ forment ensemble avec l'atome d'azote qui les porte un hétérocycle saturé,
- une guanidine cyclique de formule : 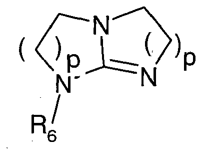 dans laquelle p est 1 ou 2, et R₆ représente un atome d'hydrogène ou un radical alkyle en C₁-C₄,
- un phosphazène de formule : 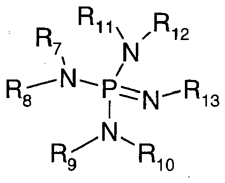
dans laquelle R7, R10, R11, et R12 représentent indépendamment un radical alkyle en C1 à C6,
R₈ et R₉ représentent indépendamment un atome d'hydrogène ou un radical alkyle en C1 à C6, ou bien R₈ et R₉ forment ensemble avec les atomes d'azote qui les portent un hétérocycle saturé,
R₁₃ représente un radical alkyle en C1 à C6.

6. Procédé de préparation de polymères selon l'une des revendications 1 à 5, dans lequel la réaction a lieu dans un solvant organique, de préférence dans un solvant halogéné ou aromatique.

7. Procédé de préparation de polymères selon l'une des revendications 1 à 6, dans lequel le solvant est un solvant halogéné, de préférence, le solvant est le dichlorométhane.

8. Procédé de préparation de polymères selon l'une des revendications 1 à 7, dans lequel l'amorceur est une amine.

9. Procédé de préparation de polymères selon l'une des revendications 1 à 7, dans lequel l'amorceur est un aminoalcool.

10. Procédé de préparation de polymères étoilés selon l'une des revendications 1 à 9, dans lequel la température de réaction est de 0 à 150°C, de préférence de 20 à 45°C.

11. Polymère de formule (I) 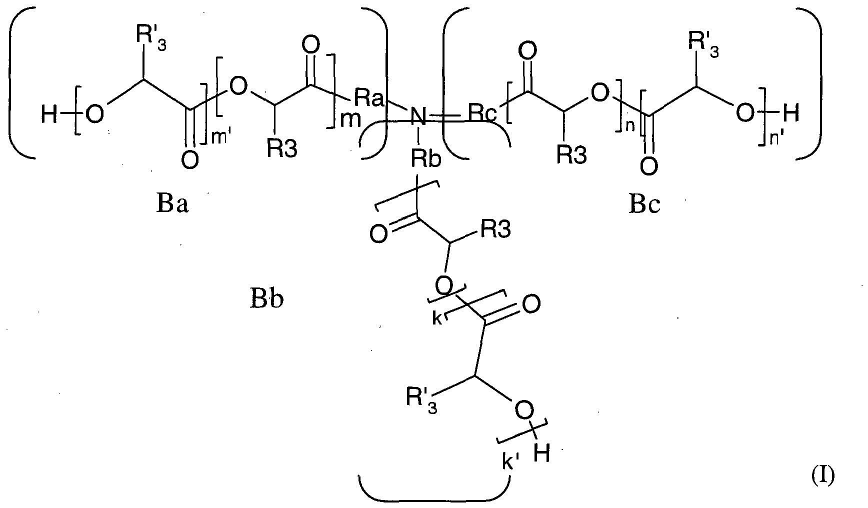 dans laquelle
n, n', m, m', k et k' représentent indépendamment un entier de 0 à 12,
Ra représente une liaison covalente, un radical alkyle en C5 à C14 linéaire ou ramifié, un radical alkylamino, un radical alkyloxy, un radical aryle ou aralkyle, étant entendu que si Ra est un radical aryle ou aralkyle alors m et m' sont nuls,
Rb représente une liaison covalente, un radical alkyle en C5 à C14 linéaire ou ramifié, un radical alkylamino ou un radical alkyloxy,
Rc représente une liaison covalente, un radical alkyle en C5 à C14 linéaire ou ramifié, un radical alkylamino, ou un radical alkyloxy,
R3 représente un atome d'hydrogène et R'3 représente un radical alkyle, étant entendu qu'au moins un des n', m' et k' est différent de zéro ;
ou bien R'3 représente un atome d'hydrogène et R3 représente un radical alkyle, étant entendu qu'au moins un des n, m et k est différent de zéro ;
et étant entendu que :
- au plus une des branches Ba, Bb et Bc représente un atome d'hydrogène
- si une des branches Ba, Bb et Bc représente l'atome d'hydrogène, alors au moins une des deux autres branches est liée à l'atome d'azote par un radical alkylamino.

12. Polymère selon la revendication 11, dans lequel R3 représente un radical alkyle, et n', m' et k' valent zéro.

13. Polymère selon la revendication 11 ou 12, dans lequel une des branches Ba, Bb et Bc représente l'atome d'hydrogène.

14. Polymère selon l'une des revendications 11 à 13, dans lequel au moins un des Ra, Rb et Rc représente un radical alkylamino.

15. Polymère selon l'une des revendications 11 à 14, dans lequel au moins un des Ra, Rb et Rc représente un radical alkyloxy.

16. Composition pharmaceutique comprenant au moins un polymère selon l'une des revendications 11 à 15.

## Patentansprüche

1. Verfahren zur Herstellung von linearen Polymeren mit Amid-Ende oder von Polymeren mit Sternarchitektur mit Amid-Zentrum durch Ringöffnung auf der Basis von Lactid- und Glycolid-Monomeren oder von einem Lactid-Monomer, umfassend die Stufen, bestehend aus:
(i) Reagierenlassen des Monomers oder der Monomeren im Überschuss mit einem Initiator in einem Lösungsmittel, wobei der Initiator aus einem Amin und einem Aminoalkohol ausgewählt ist, mit der Maßgabe, dass der Initiator wenigstens eine primäre oder sekundäre Amin-Funktion aufweist;
(ii) Zusetzen eines Katalysators, wobei der Katalysator eine nicht-nukleophile Base ist und wenigstens ein neutrales Stickstoffatom des sp²-Typs umfasst;
(iii) Neutralisieren des Reaktionsgemisches.

2. Verfahren zur Herstellung von Polymeren nach Anspruch 1, wobei das Monomer das Lactid ist.

3. Verfahren zur Herstellung von Polymeren nach Anspruch 1, wobei die Polymere auf der Basis eines Lactid-Monomers und eines Glycolid-Monomers hergestellt werden.

4. Verfahren zur Herstellung von Polymeren nach einem der Ansprüche 1 bis 3, wobei die Stufe (ii) nach vollständiger Einarbeitung des Initiators durchgeführt wird.

5. Verfahren zur Herstellung von Polymeren nach einem der Ansprüche 1 bis 4, wobei der basische Katalysator ausgewählt wird aus:
- 1,8-Diazabicyclo{5.4.0]undec-7-en,
- 1,5-Diazabicyclo[4.3.0]non-5-en,
- einer 4-Aminopyridin-Verbindung der Formel: 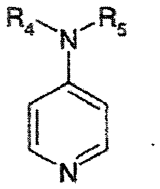 in der R₄ und R₅ unabhängig ausgewählt sind aus einem Wasserstoffatom oder einem C₁-C₁₂-Alkylrest, oder auch R₄ und R₅ zusammen mit dem Stickstoffatom, das sie trägt, einen gesättigten Heterocyclus bilden,
- einem cyclischen Guanidin der Formel: 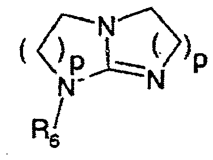 in der p 1 oder 2 ist und R₆ ein Wasserstoffatom oder einen C₁-C₄-Alkylrest darstellt,
- einem Phosphazen der Formel: 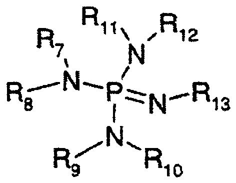
in der R₇, R₁₀, R₁₁ und R₁₂ unabhängig einen C₁-C₆-Alkylrest darstellen,
R₈ und R₉ unabhängig ein Wasserstoffatom oder einen C₁-C₆-Alkylrest darstellen, oder auch R₈ und R₉ zusammen mit den Stickstoffatomen, die sie tragen, einen gesättigten Heterocyclus bilden,
R₁₃ einen C₁-C₆-Alkylrest darstellt.

6. Verfahren zur Herstellung von Polymeren nach einem der Ansprüche 1 bis 5, wobei die Reaktion in einem organischen Lösungsmittel, vorzugsweise in einem halogenierten oder aromatischen Lösungsmittel stattfindet.

7. Verfahren zur Herstellung von Polymeren nach einem der Ansprüche 1 bis 6, wobei das Lösungsmittel ein halogeniertes Lösungsmittel ist, das Lösungsmittel vorzugsweise Dichlormethan ist.

8. Verfahren zur Herstellung von Polymeren nach einem der Ansprüche 1 bis 7, wobei der Initiator ein Amin ist.

9. Verfahren zur Herstellung von Polymeren nach einem der Ansprüche 1 bis 7, wobei der Initiator ein Aminoalkohol ist.

10. Verfahren zur Herstellung von Sternpolymeren nach einem der Ansprüche 1 bis 9, wobei die Reaktionstemperatur 0 bis 150 °C, vorzugsweise 20 bis 45 °C, ist.

11. Polymer der Formel (I) 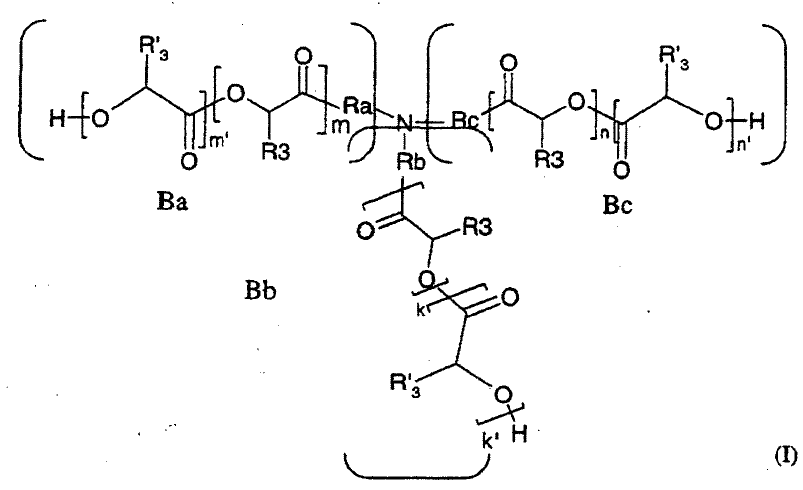 in der
n, n', m, m', k und k' unabhängig eine ganze Zahl von 0 bis 12 darstellen,
Ra eine kovalente Bindung, einen linearen oder verzweigten C₅-C₁₄-Alkylrest, einen Alkylaminorest, einen Alkyloxyrest, einen Aryl- oder Aralkylrest darstellt, mit der Maßgabe, dass, wenn Ra ein Aryl- oder Aralkylrest ist, dann m und m' Null sind;
Rb eine kovalente Bindung, einen linearen oder verzweigten C₅-C₁₄-Alkyl-rest, einen Alkylaminorest oder einen Alkyloxyrest darstellt;
Rc eine kovalente Bindung, einen linearen oder verzweigten C₅-C₁₄-Alkylrest, einen Alkylaminorest oder einen Alkyloxyrest darstellt;
R3 ein Wasserstoffatom darstellt und R'3 einen Alkylrest darstellt, mit der Maßgabe, dass wenigstens eines von n', m' und k' von Null verschieden ist;
oder R'3 ein Wasserstoffatom darstellt und R3 einen Alkylrest darstellt, mit der Maßgabe, dass wenigstens eines von n, m und k von Null verschieden ist;
und mit der Maßgabe, dass:
- höchstens eine der Verzweigungen Ba, Bb und Bc ein Wasserstoffatom darstellt,
- wenn eine der Verzweigungen Ba, Bb und Bc ein Wasserstoffatom darstellt, dann wenigstens eine der zwei anderen Verzweigungen durch einen Alkylaminorest an das Stickstoffatom gebunden ist.

12. Polymer nach Anspruch 11, wobei R3 einen Alkylrest darstellt und n', m' und k' den Wert Null haben.

13. Polymer nach Anspruch 11 oder 12, wobei eine der Verzweigungen Ba, Bb und Bc ein Wasserstoffatom darstellt.

14. Polymer nach einem der Ansprüche 11 bis 13, wobei wenigstens eines von Ra, Rb und Rc einen Alkylaminorest darstellt.

15. Polymer nach einem der Ansprüche 11 bis 14, wobei wenigstens eines von Ra, Rb und Rc einen Alkyloxyrest darstellt.

16. Pharmazeutische Zusammensetzung, die wenigstens ein Polymer nach einem der Ansprüche 11 bis 15 umfasst.

## Claims

1. Method for the preparation of linear polymers with an amide end or with star architecture with an amide core by ring opening based on lactide and glycolide monomers or a lactide monomer, comprising the steps consisting of:
(i) reacting the monomer or monomers in excess with an initiator in a solvent, said initiator being chosen from an amine and an amino alcohol, given that the initiator has at least one primary or secondary amine function,
(ii) adding a catalyst, said catalyst being a non-nucleophilic base, and comprising at least one neutral nitrogen atom of sp2 type,
(iii) neutralizing the reaction mixture.

2. Method for the preparation of polymers according to claim 1, in which the monomer is lactide.

3. Method for the preparation of polymers according to claim 1, in which the polymers are prepared based on a lactide monomer and a glycolide monomer.

4. Method for the preparation of polymers according to one of claims 1 to 3, in which step (ii) is carried out after the complete incorporation of the initiator.

5. Method for the preparation of polymers according to one of claims 1 to 4, in which the basic catalyst is chosen from:
- 1,8-diazabicyclo[5.4.0]undec-7-ene,
- 1,5-diazabicyclo[4.3.0]non-5-ene,
- a 4-amino-pyridine compound of formula: 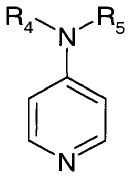 in which R₄ and R₅ are independently chosen from a hydrogen atom or a C₁-C₁₂ alkyl radical; or R₄ and R₅ form together with the nitrogen atom which bears them a saturated heterocycle,
- a cyclic guanidine of formula: 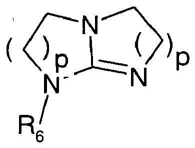 in which p is 1 or 2, and R₆ represents a hydrogen atom or a C1-C4 alkyl radical,
- a phosphazene of formula: 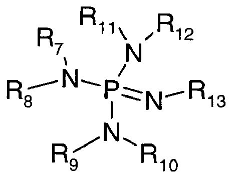
in which R7, R10, R 11, and R 12 represent independently a C1 to C6 alkyl radical,
R₈ and R₉ represent independently a hydrogen atom or a C1 to C6 alkyl radical, or R₈ and R₉ form together with the nitrogen atoms which bear them a saturated heterocycle,
R₁₃ represents a C1 to C6 alkyl radical.

6. Method for the preparation of polymers according to one of claims 1 to 5, in which the reaction takes place in an organic solvent, preferably in a halogenated or aromatic solvent.

7. Method for the preparation of polymers according to one of claims 1 to 6, in which the solvent is a halogenated solvent, preferably, the solvent is dichloromethane.

8. Method for the preparation of polymers according to one of claims 1 to 7, in which the initiator is an amine.

9. Method for the preparation of polymers according to one of claims 1 to 7, in which the initiator is an amino alcohol.

10. Method for the preparation of star polymers according to one of claims 1 to 9, in which the reaction temperature is from 0 to 150°C, preferably from 20 to 45°C.

11. Polymer of formula (I) 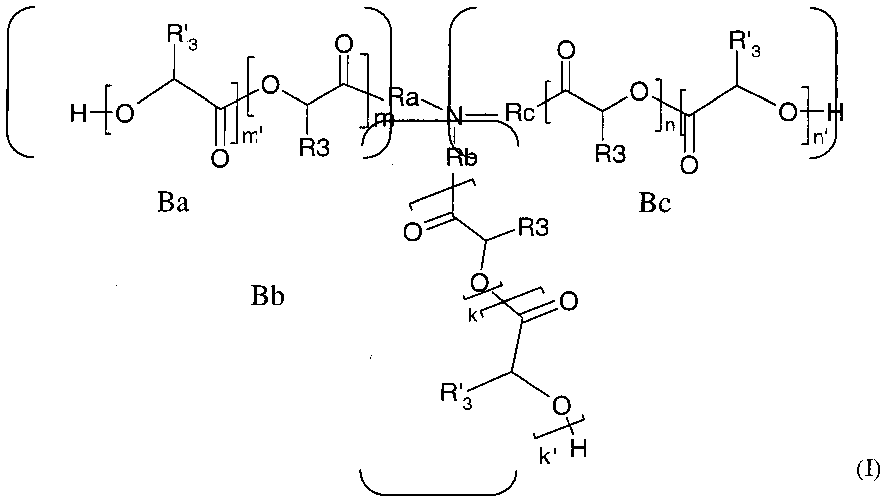 in which
n, n', m, m', k and k' represent independently an integer from 0 to 12,
Ra represents a covalent bond, a linear or branched C5 to C14 alkyl radical, an alkylamino radical, an alkyloxy radical, an aryl or aralkyl radical, it being understood that if Ra is an aryl or aralkyl radical then m and m' are zero,
Rb represents a covalent bond, a linear or branched C5 to C14 alkyl radical, an alkylamino radical or an alkyloxy radical,
Rc represents a covalent bond, a linear or branched C5 to C14 alkyl radical, an alkylamino radical, or an alkyloxy radical,
R3 represents a hydrogen atom and R'3 represents an alkyl radical, it being understood that at least one of the n', m' and k' is different from zero;
or R'3 represents a hydrogen atom and R3 represents an alkyl radical, it being understood that at least one of the n, m and k is different from zero;
and it being understood that:
- at most one of the branches Ba, Bb and Bc represents a hydrogen atom
- if one of the branches Ba, Bb and Bc represents the hydrogen atom, then at least one of the other two branches is linked to the nitrogen atom by an alkylamino radical.

12. Polymer according to claim 11, in which R3 represents an alkyl radical, and n', m' and k' are zero.

13. Polymer according to claim 11 or 12, in which one of the branches Ba, Bb and Bc represents the hydrogen atom.

14. Polymer according to one of claims 11 to 13, in which at least one of Ra, Rb and Rc represents an alkylamino radical.

15. Polymer according to one of claims 11 to 14, in which at least one of Ra, Rb and Rc represents an alkyloxy radical.

16. Pharmaceutical composition comprising at least one polymer according to one of claims 11 to 15.
